# EUROPEAN PATENT SPECIFICATION

(11) **EP 2 324 833 B1**
(45) Date of publication and mention of the grant of the patent: **19.07.2017**
(21) Application number: 09809530.0
(22) Date of filing: 25.08.2009
(51) Int. Cl.: A61K 31/451, A61K 9/70, A61K 47/10, A61K 47/38, A61P 1/12, A61K 31/045, A61K 9/00

(54) **FILM PREPARATION CONTAINING LOPERAMIDE HYDROCHLORIDE**
FILMPRÄPARAT MIT LOPERAMID-HYDROCHLORID
PRÉPARATION DE FILM CONTENANT DU CHLORHYDRATE DE LOPÉRAMIDE

(30) Priority: 25.08.2008 JP 2008214839
(43) Date of publication of application: 25.05.2011
(73) Proprietor: Kyukyu Pharmaceutical Co., Ltd., Tokyo 103-0023 (JP); Kowa Company, Ltd., Nagoya-shi, Aichi-ken 460-8625 (JP)
(72) Inventor: AWAMURA, Tsutomu, Imizu-shi Toyama 939-0351 (JP); NISHIKAWA, Hisanobu, Imizu-shi Toyama 939-0351 (JP); INAGI, Toshio, Fuji-shi Shizuoka 417-8650 (JP)
(74) Representative: Hartz, Nikolai
(86) International application number: PCT/JP2009/004087
(87) International publication number: WO 2010/023874

(56) References cited:
- WO-A1-03/070227
- WO-A2-2008/039737
- AU-B2- 771 862
- JP-A- 2005 232 072
- JP-A- 2005 263 704
- JP-A- 2005 289 867
- JP-A- 2007 254 340
- JP-T- 2002 525 306
- JP-T- 2003 527 410
- JP-T- 2008 517 935
- YASUHIKO NAKAMURA: 'Nigami no Masking to Joho Seizai' JOURNAL OF PHARMACEUTICAL SCIENCE AND TECHNOLOGY, JAPAN vol. 65, no. 3, 2005, pages 159 - 164, XP008142946

## Description

### [Technical Field]

The present invention relates to a film preparation in which a bitter taste of loperamide hydrochloride is masked, a method for producing the same, and a method for masking the bitter taste in the film preparation containing loperamide hydrochloride.

### [Background Art]

Loperamide hydrochloride is widely used as an antidiarrheal. For example, preparations containing loperamide hydrochloride are already marketed, which can be taken if one is acutely stricken with diarrhea in a situation where taking with water is difficult, such as during commuting or meeting. However, as loperamide hydrochloride has a very strong bitter taste, there is a problem in the sensation when it is taken.

As an example of a method for masking such a bitter taste, Patent Documents 1 to 5 propose that a preparation contains a drug having a bitter taste together with a sweetener, a surfactant and the like. However, none of such known art suggests or teaches how to mask a bitter taste in a film preparation containing loperamide hydrochloride.

As another method for masking a bitter taste in a drug, it has been proposed to add a drug having a bitter taste as well as 0.1 to 2.25% by weight of menthol in a solid pharmaceutical composition internally taken by dissolving in the oral cavity or by mastication (Patent Document 6). However, virtually no dosage form other than tablet is disclosed and there has been no suggestion or teaching about masking of a bitter taste in a film preparation containing loperamide hydrochloride.

### [Prior Art]

### [Patent Document]

[Patent Document 1] JP-A-5-117149
[Patent Document 2] JP-A-9-30969
[Patent Document 3] JP-A-9-323931
[Patent Document 4] JP-A-10-101582
[Patent Document 5] JP-A-11-35450
[Patent Document 6] JP-A-2000-95707

WO 03/070227 relates to a taste-masked film-type or wafter-type medicinal preparation that may contain loperamide hydrochloride as active ingredient. The preparation may contain a blood flow stimulator such as menthol. The preparation may have a bilayer or multi-layer structure, one layer containing a mucoadhesive or bio-adhesive substance for adhesion to mucosa.

WO 2008/039737 relates to a method of administering a film product containing a drug.

AU 771 862 B2 relates to a fast dissolving orally consumable film.

### [Summary of Invention]

### [Problems to be solved by the Invention]

The present invention is to provide a film preparation in which a bitter taste of loperamide hydrochloride is masked.

### [Means for Solving the Problem]

The inventors of the present invention conducted various researches to achieve the foregoing object. As a result, they surprisingly found that a bitter taste of loperamide hydrochloride could be noticeably masked by producing a film preparation in which coating layers containing specific ingredients were laminated on both sides of a drug-containing layer containing loperamide hydrochloride and specific ingredients according to claim 1.

Specifically, the present invention provides a film preparation including: a drug-containing intermediate layer containing loperamide hydrochloride, a terpene and a film-forming agent; and coating layers containing a film-forming agent and a plasticizer (but not a terpene) and laminated on both sides of the drug-containing intermediate layer.

The present invention also provides a method for masking a bitter taste in a film preparation containing loperamide hydrochloride, including
laminating coating layers containing a film-forming agent and a plasticizer (but not a terpene) on both sides of a drug-containing intermediate layer containing loperamide hydrochloride, a terpene and a film-forming agent.

The present invention further provides a method for producing a film preparation containing loperamide hydrochloride, in which a bitter taste derived from loperamide hydrochloride is masked, including
laminating coating layers containing a film-forming agent and a plasticizer (but not a terpene) on both sides of a drug-containing intermediate layer containing loperamide hydrochloride, a terpene and a film-forming agent.

### [Effects of the Invention]

The film preparation of the present invention can reduce uncomfortable sensation when it is orally taken, because a bitter taste derived from loperamide hydrochloride is masked. As the film preparation of the present invention is rapidly disintegrated and dissolved (i.e., has a rapidly dissolving property) only with moisture in the oral cavity, it begins to work quickly and is also very convenient when it is carried and taken. The film preparation of the present invention is therefore superior in rapid relief of diarrhea symptoms, such as diarrhea due to excess eating and drinking, diarrhea due to getting chilled while asleep, and diarrhea accompanied by abdominal pain and the like, due to an excellent antidiarrheal effect of loperamide hydrochloride.

### [Mode for Carrying Out the Invention]

A film preparation of the present invention includes: a drug-containing intermediate layer containing loperamide hydrochloride; and coating layers provided on both sides thereof. In the film preparation of the present invention, the drug-containing intermediate layer further contains a terpene and a film-forming agent, and the coating layer contains a film-forming agent and a plasticizer but does not contain a terpene.

First, components in the film preparation according to the present invention are described.

The dose of loperamide hydrochloride in the film preparation of the present invention can be selected as appropriate. For example, it is preferred for adults that the dose for one treatment is 0.5 to 2 mg and the daily dose is 1 to 4 mg. It is more preferred that the dose for one treatment is 0.5 to 1 mg and the daily dose is 1 to 2 mg. Thus, for adults, the daily dose can be orally taken as one dose or can be divided into several doses. The dose can also be adjusted depending on the age, body weight, or symptom as appropriate.

The content of loperamide hydrochloride in the film preparation of the present invention is preferably 0.1% to 15% by weight to the whole film preparation, more preferably 0.5% to 10% by weight, particularly preferably 1% to 5% by weight. As a particularly preferred embodiment, it is preferred to add preferably 1% to 5% by weight, particularly preferably 2% to 4% by weight of loperamide hydrochloride into the drug-containing intermediate layer.

In the present invention, the term "terpene" represents a concept of terpenes and essential oils containing terpenes. Specific examples of terpenes include limonene, pinene, camphene, cymene, cineol, citronellol, geraniol, nerol, linalool, menthol, terpiol, rhodinol, borneol, isoborneol, menthone, camphor, eugenol, and cinnzeylanol and the like. These terpenes have a single stereoisomer and a racemate. Examples of essential oils containing terpenes include orange peel oil, orange oil, mentha oil, white camphor oil, eucalyptus oil, turpentine oil, lemon oil, ginger oil, clove oil, cinnamon oil, lavender oil, fennel oil, chamomile oil, perilla oil, and spearmint oil and the like. One or a combination of two or more of these terpenes may be used.

In the present invention, terpenes are preferably menthol and mentha oil.

The content of the terpene in the film preparation of the present invention is preferably 0.2% to 15% by weight to the whole film preparation, more preferably 0.5% to 10% by weight, more preferably 1% to 5% by weight, particularly preferably 1% to 3% by weight. As a particularly preferred embodiment, it is preferred to add preferably 1% to 5% by weight, more preferably 2% to 4% by weight of the terpene into the drug-containing intermediate layer. It is particularly preferred that the content of the terpene is a nearly equal amount to that of loperamide hydrochloride.

In the present invention, the term "film-forming agent" means an agent having a property of forming a film when an aqueous solution of the agent is dried. The film-forming agents are not particularly limited so long as they are materials which have such a film-forming ability. Examples thereof include hypromellose (hydroxypropylmethylcellulose), hydroxypropylcellulose, pullulan, hydroxyethylcellulose, sodium carboxymethylcellulose, calcium carboxymethylcellulose, potassium carboxymethylcellulose, carboxymethylcellulose, polyvinylpyrrolidone, and sodium alginate and the like. One or a combination of two or more thereof selected from these film-forming agents is preferred. Above all, hypromellose, hydroxypropylcellulose, and the like are more preferred. It is particularly preferred to add hypromellose into the coating layer and add hydroxypropylcellulose into the drug-containing intermediate layer.

The hypromellose used herein means a mixed ether of methyl and hydroxypropyl of cellulose and can be produced by known methods. Further, as hypromellose, commercially available products (e.g., those produced by Shin-Etsu Chemical Co., Ltd., Dow Chemical Japan Ltd., and Matsumoto Yushi-Seiyaku Co., Ltd. and the like) may be used. The substitution degree of a methoxy group and a hydroxypropoxy group in hypromellose is not particularly limited. Hypromellose with a desired substitution degree can be obtained by presetting the substitution degree before etherifying cellulose. In the present invention, hypromellose preferably contains 10% to 50%, more preferably 16.5% to 30%, particularly preferably 25% to 30% of a methoxy group and preferably 2% to 35%, more preferably 4% to 32%, particularly preferably 4% to 20% of a hydroxypropoxy group. Above all, hypromellose containing 16.5% to 30% of a methoxy group and 4% to 32% of a hydroxypropoxy group is particularly preferred. Further, hypromellose containing 25% to 30% of a methoxy group and 4% to 20% of a hydroxypropoxy group is more preferred. Among commercially available products, Hypromellose 1828, Hypromellose 2208, Hypromellose 2906, and Hypromellose 2910 are preferred. The viscosity of hypromellose is not particularly limited but, for example, the kinematic viscosity of a 2% aqueous solution at 20°C (Japanese Pharmacopoeia 15th Edition) is preferably approx. 6 mPa·s.

Meanwhile, hydroxypropylcellulose means a hydroxypropyl ether of cellulose and can be produced by known methods. As hydroxypropylcellulose, commercially available products (e.g., those produced by San-Ei Gen F.F.I, Inc, Nippon Soda Co., Ltd. and the like) may be used. The substitution degree in hydroxypropylcellulose is not particularly limited. Hydroxypropylcellulose with a desired substitution degree can be obtained by presetting the substitution degree before etherifying cellulose. In the present invention, hydroxypropylcellulose preferably contains 50% to 80%, more preferably 53.4% to 77.5% of a hydroxypropoxy group. The viscosity of hydroxypropylcellulose is not particularly limited but, for example, the kinematic viscosity in a 2% aqueous solution of hydroxypropylcellulose at 20°C (Japanese Pharmacopoeia 15th Edition) is preferably 2.0 to 2.9 mPa·s.

The content of the film-forming agent in the film preparation of the present invention is preferably 40% to 95% by weight to the whole film preparation, more preferably 45% to 80% by weight, particularly preferably 50% to 65% by weight. As a particularly preferred embodiment, preferably 68% to 80% by weight, particularly preferably 70% to 75% by weight of the film-forming agent is added into the coating layer and preferably 40% to 50% by weight, particularly preferably 45% to 50% by weight thereof is added into the drug-containing intermediate layer.

In the present invention, a plasticizer means a compound which is compatible with the film-forming agent and imparts flexibility to the film-forming agent. The plasticizers are not particularly limited so long as they are materials having such a plasticizing ability. Examples thereof include glycerin, sesame oil, sorbitol, castor oil, propylene glycol, polyoxyethylene polyoxypropylene glycol, polysorbate 80 (polyoxyethylene (20) sorbitan oleic acid ester), and polyethylene glycol [e.g., macrogol 400 (n [polymerization degree in oxyethylene unit, similarly hereunder] = 7 to 9), macrogol 600 (n = 11 to 13), macrogol 1500 (an equivalent mixture of n = 5 to 6 with n = 28 to 36), macrogol 4000 (n = 59 to 84), and macrogol 6000 (n = 165 to 210)] and the like. As plasticizers, one or a combination of two or more thereof selected from the plasticizers is preferred. Above all, glycerin, propylene glycol, and macrogol 400 are more preferred.

The content of the plasticizer is preferably 1% to 20% by weight to the whole film preparation, more preferably 3% to 15% by weight, particularly preferably 5% to 10% by weight. As a particularly preferred embodiment, preferably 5% to 10% by weight, particularly preferably 7% to 10% by weight of the plasticizer is added into each of the coating layer and the drug-containing intermediate layer.

In addition to loperamide hydrochloride, the film preparation of the present invention may contain one or two or more of medicine such as antacid, stomachic, digestive, drug for controlling intestinal function, antidiarrheal other than loperamide hydrochloride, analgesic anticonvulsant, vitamin, amino acid and other crude drug and the like.

Examples of antacid include dry aluminium hydroxide gel, magnesium aluminosilicate, magnesium aluminometasilicate, aluminium silicate, hydrotalcite, magnesia alumina hydrate, an aluminium hydroxide gel, coprecipitation products of aluminium hydroxide and sodium hydrogencarbonate, dry mixed gel of aluminium hydroxide and magnesium carbonate, coprecipitation products of aluminium hydroxide, calcium carbonate, and magnesium carbonate, magnesium carbonate, magnesium oxide, magnesium hydroxide, magnesium silicate, coprecipitation products of magnesium hydroxide and aluminium potassium sulfate, anhydrous calcium hydrogen phosphate, calcium hydrogen phosphate, precipitated calcium carbonate, calcium lactate, calcium hydroxide, sodium hydrogencarbonate, sodium citrate, sodium acetate, anion-exchange resins such as polyaminomethylene resins, H2 receptor antagonists such as famotidine, ranitidine, and cimetidine, pirenzepine hydrochloride, proton pump inhibitors, gastric mucin, cuttlebone, abalone shell, oyster shell, aminoacetic acid, dihydroxyaluminium aminoacetate and scopolia extract and the like.

Examples of stomachic include crude drugs such as anise seed, aloe, fennel, turmeric, lindera root, Rabdosia japonica, scutellaria root, Plectranthi herba, coptis rhizome, processed garlic, zedoary, Pogostemoni Herb, cinchona, Nux vomica, ginger, calmus root, processed ginger, trifoliate orange, immature orange, cinnamon bark, gentian, red ginseng, magnolia bark, evodia fruit, pepper, calumba, condurango, zanthoxylum fruit, Hedychium spicatum, Perillae fructus, amomum seed, Cardamomi Fructus, unripe orange fruit peel, gypsum root, centaurium plant, Swertia Harb, atractylodes lancea rhizome, perilla herb, star anise, rhubarb, Panax japonicus rhizome, clove, citrus unshiu peel, capsicum, bitter orange peel, animal bile, Picrasma wood, nutmeg, ginseng, mentha herb, Piper longum, atractylodes rhizome, hop, Nux vomica extract, Menyanthes trifoliata, saussurea root, Bitter Cardamon, Japanese gentian, alpinia officinarum rhizome, and lemon and the like, betaine hydrochloride, glutamic acid hydrochloride, carnitine chloride, bethanechol chloride, dry yeast, and trimebutine maleate and the like.

Examples of digestive include diastase, pancreatin, pepsin, ptyalin, β-galactosidase, amylase, trypsin, papain, protease, lipase, cellulase, mamitase, samprose, newlase, Prozyme, molsin, panprosin, biotamilase, hirotase, ursodeoxycholic acid, oxycholanic acid, cholic acid, bile powder, bile extract, dihydrocholic acid, and animal bile and the like.

Examples of drug for controlling intestinal function include intestine-regulating viable bacterial components, Mallotus Bark, gambir, Nume fructus, Cassia Seed, and Geranium Herb and the like.

Examples of antidiarrheal other than loperamide hydrochloride include acrinol, berberine chloride, guaiacol, creosote, phenyl salicylate, guaiacol carbonate, berberine tannate, bismuth subsalicylate, bismuth subnitrate, bismuth subcarbonate, bismuth subgallate, tannic acid, albumin tannate, methylene thymol tannin, kaolin, natural aluminium silicate, aluminium hydroxynaphthoate, pectin, medicinal charcoal, precipitated calcium carbonate, calcium lactate, calcium hydrogen phosphate, gambir, Nume fructus, Phellodendron bark, Coptis rhizome, sophora root, Geranium herb, Rhus chinensis, crataegus, Swertia Herb, and Myricae cortex and the like.

Examples of analgesic anticonvulsant include papaverine hydrochloride, ethyl aminobenzoate, scopolamine hydrobromide, methylscopolamine bromide, corydalis tuber, glycyrrhiza, magnolia bark, peony root, timepidium bromide, oxyphencyclimine hydrochloride, dicyclomine hydrochloride, metixene hydrochloride, methylatropine bromide, methyl bromide -1-hyoscyamine, methylbenactyzium bromide, belladonna extract, scopolia extract, diphenylpiperidinomethyldioxolane iodide, total alkaloid citrate of scopolia root, isopropamide iodide, anisotropine methylbromide, scopolamine butylbromide, tiquizium bromide, and oxethazaine and the like.

Examples of vitamin include vitamin A such as retinal, retinol, retinoic acid, carotene, dehydroretinal, lycopene, and pharmacologically acceptable salts thereof, vitamin B such as thiamine, thiamine disulfide, dicethiamine, octothiamine, cycothiamine, bisibutiamine, bisbenthiamine, prosultiamine, benfotiamine, fursultiamine, riboflavin, flavin adenine dinucleotide, pyridoxine, pyridoxal, hydroxocobalamin, cyanocobalamin, methylcobalamin, deoxyadenocobalamin, folic acid, tetrahydrofolic acid, dihydrofolic acid, nicotinic acid, nicotinic acid amide, nicotinic alcohol, pantothenic acid, panthenol, biotin, choline, inositol, and pharmacologically acceptable salts thereof, vitamin C such as ascorbic acid, erythorbic acid, derivatives thereof, and pharmacologically acceptable salts thereof, vitamin D such as ergocalciferol, cholecalciferol, hydroxycholecalciferol, dihydroxycholecalciferol, dihydrotachysterol, and pharmacologically acceptable salts thereof, vitamin E such as tocopherol and derivatives thereof, ubiquinone derivatives, and pharmacologically acceptable salts thereof, hesperidin, carnitine, ferulic acid, γ-oryzanol, orotic acid, rutin, eriocitrin, and pharmacologically acceptable salts thereof and the like.

Examples of amino acid include leucine, isoleucine, valine, methionine, threonine, alanine, phenylalanine, tryptophan, lysine, asparagine, aspartic acid, serine, glutamine, glutamic acid, proline, tyrosine, cysteine, histidine, ornithine, hydroxyproline, hydroxylysine, aminoethylsulfonic acid, and pharmaceutically acceptable salts thereof and the like.

Examples of other crude drug include ginseng, coix seed, chamomile, cinnamon bark, kakkonto, ephedra herb, Nandinae fructus, Pruni jamasakura cortex, glycyrrhiza, licorice, apricot kernel, plantago seed, plantago herb, Lycoris radiata, senega, ipecac, Fritillaria bulb, gambir, fennel, scutellaria root, Trichosanthis semen, cinnamon bark, oriental bezoar, schisandra fruit, asiasarum root, Asteris radix, musk, Adenophrae radix, ginger, mulberry bark, perilla herb, Panax japonicius rhizome, citrus unshiu peel, ophiopogon tuber, pinellia tuber, and Mallotus bark and the like.

Further, one or two or more of drug additives usually used may be used in the film preparation of the present invention, if necessary. Examples of drug additives include, but are not limited to, disintegrating agent, filler, poorly water-soluble polymeric substance, colorant, antioxidant, taste-masking agent and flavoring agent and the like.

Examples of disintegrating agent include carmellose and a salt thereof, starch, sucrose fatty acid esters, gelatin, sodium hydrogencarbonate, dextrin, dehydroacetic acid and salts thereof, povidone, polyoxyethylene hydrogenated castor oil 60, and polyoxyethylene polyoxypropylene glycol and the like.

Examples of filler include inorganic filler such as titanium oxide, alumina magnesium hydroxide, magnesium hydroxide, aluminium silicate, silicon dioxide, anhydrous sodium sulfate, anhydrous calcium hydrogen phosphate, sodium chloride, hydrous amorphous silicon oxide, magnesium aluminate silicate, calcium silicate, magnesium silicate, light anhydrous silicic acid, heavy anhydrous silicic acid, magnesium oxide, calcium sulfate, calcium monohydrogen phosphate, calcium hydrogen phosphate, sodium hydrogen phosphate, potassium dihydrogen phosphate, calcium dihydrogen phosphate, and sodium dihydrogen phosphate and organic filler such as maltose syrup powder, starch, fructose, caramel, agar, xylitol, paraffin, cellulose, sorbitol, sucrose, maltose, lactose, white soft sugar, glucose, Pullulan, polyoxyethylene hydrogenated castor oil, maltitol, hydrogenated maltose starch syrup, powdered hydrogenated maltose starch syrup powder, sorbitol, erythritol, mannitol, lactitol, trehalose and hydrogenated palatinose.

Examples of poorly water-soluble polymeric substance include ethylcellulose, hydroxypropylmethylcellulose phthalate, methylcellulose, polyvinyl alcohol, and carboxyvinyl polymer and the like.

Examples of colorant include yellow ferric oxide, brown ferric oxide, caramel, black ferric oxide, titanium oxide, ferric oxide, tar dye, aluminium lake dye, and sodium copper chlorophyllin and the like.

Examples of flavoring agent include orange flavor, grapefruit flavor, strawberry flavor, and lemon flavor and the like.

Examples of antioxidant include ascorbic acid, sodium hydrogen sulfite, sodium sulfite, sodium edetate, erythorbic acid, tocopherol acetate, dibutylhydroxytoluene, natural vitamin E, tocopherol, and butylhydroxyanisole and the like.

Examples of taste-masking agent include acidulant such as ascorbic acid, tartaric acid, citric acid, malic acid, and salts thereof and sweeteners such as aspartame, stevia, sucralose, glycyrrhizinic acid, thaumatin, acesulfame potassium, saccharin, and saccharin sodium and the like.

It is preferred to add 1% to 8% by weight to the whole film preparation, particularly 2% to 6% by weight of the disintegrating agent. As a particularly preferred embodiment, preferably 5% to 20% by weight, particularly 7% to 15% by weight of the disintegrating agent is added into a coating layer.

It is preferred to add 15% to 60% by weight to the whole film preparation, particularly preferably 15% to 30% by weight of the filler. As a particularly preferred embodiment, preferably 5% to 20% by weight, particularly preferably 7% to 15% by weight of the filler is added into a coating layer and preferably 10% to 40% by weight, particularly preferably 25% to 35% by weight of the filler is added into a drug-containing intermediate layer.

The poorly water-soluble polymeric substance preferably in the range of 1% to 10% by weight can be added to the whole film preparation. The Colorant preferably in the range of 0.05% to 10% by weight can be added to the whole film preparation. Antioxidant preferably in the range of 0.1% to 5% by weight can be added to the whole film preparation. Taste-masking agent preferably in the range of 1% to 10% by weight can be added to the whole film preparation. Flavoring agent preferably in the range of 0.01% to 0.1% by weight can be added to the whole film preparation.

The film preparation of the present invention has a basic form composed of a three-layer structure in which a coating layer, a drug-containing intermediate layer and a coating layer are laminated in this order. When the same type of layers are laminated adjacent to each other, they are attached and integrated to each other to have the same function. In the present invention, these layers are therefore regarded as substantially one layer. The thickness of the whole film preparation is preferably 30 to 300 µm, particularly preferably 30 to 200 µm. In this case, the thickness of the coating layer is preferably 5 to 100 µm, particularly preferably 10 to 50 µm. The thickness of the drug-containing intermediate layer is preferably 10 to 200 µm, particularly preferably 10 to 100 µm. Because of these thicknesses, the preparation can be rapidly dissolved in the oral cavity. Further, the size of the film preparation of the present invention is not particularly limited so long as it is easily taken. For example, a size of approx. 0.5 to 10 cm² is preferred. The shape is not particularly limited either so long as it is easily taken. For example, a rectangular, circular, or elliptical shape or the like can be suitably selected.

Further, in the film preparation of the present invention, a functional layer may be provided between the drug-containing intermediate layer and the coating layer so long as achievement of the object of the present invention is not precluded. Examples of such a layer include a support layer for improving a handling property which contains hydroxypropyl methylcellulose as a main component and a moisture prevention layer for preventing moisture which contains ethylcellulose as a main component. The thickness of each layer can be suitably selected within the range which does not preclude achievement of the object of the present invention.

The film preparation of the present invention can be suitably prepared by common or known methods. For example, the film preparation of the present invention can be prepared by uniformly applying a first coating layer on a release film made of polyethylene terephthalate (PET) or the like, uniformly applying a drug-containing intermediate layer thereon, and then uniformly applying a second coating layer on the intermediate layer. Further, the film preparation of the present invention can also be prepared by separately preparing a first interim product obtained by uniformly applying the first coating layer on the release film and further uniformly applying the intermediate drug layer thereon and a second interim product prepared by uniformly applying the second coating layer on the release film and further uniformly applying the intermediate drug layer thereon, and then contacting the drug intermediate layers of both interim products so that they are opposed to each other and laminating them by pressure. The film-forming agents in the first coating layer and the second coating layer may be identical to or different from each other.

### [Examples]

Hereafter, the present invention will be specifically described with reference to the examples and the like. However, the scope of the present invention is not limited to the examples described below.

### Example 1

50 g of powdered hydrogenated maltose starch syrup and 40 g of macrogol 400 were dissolved in 500 g of water. Then, to this solution was added a solution obtained by dispersing 50 g of titanium oxide in 750 g of anhydrous ethanol. Then, 360 g of hypromellose was added to the resulting solution to obtain a solution for preparing a coating layer.

27 g of loperamide hydrochloride, 64.8 g of macrogol 400, 256.5 g of powdered hydrogenated maltose starch syrup, 54 g of saccharin sodium, and 27 g of 1-menthol were dissolved in a mixture solution of 810 g of water and 810 g of anhydrous ethanol. To this mixture solution were added 380.7 g of hydroxypropylcellulose and 1.35 g of a flavoring agent to obtain a solution for preparing a drug-containing intermediate layer.

The solution for preparing the coating layer was uniformly applied on a PET film and then dried with hot air to form a coating layer with a weight of 5 mg per area of 2.72 cm². The solution for preparing the drug-containing intermediate layer was uniformly applied on the upper side of the coating layer and then dried with hot air to form a drug-containing intermediate layer with a weight of 7.5125 mg per area of 2.72 cm² as Interim Product 1.

Two sets of Interim Product 1 were prepared, contacted so that the drug-containing intermediate layers are opposed to each other, and bonded by pressure, to obtain Interim Product 2 in which the coating layer, the drug-containing intermediate layer and the coating layer were laminated in this order between the two PET films.

One PET film of Interim Product 2 was peeled off, the product was cut into an area of 2.72 cm², and the other PET film was peeled off to obtain the film preparation of the present invention.

### Comparative Example 1

50 g of powdered hydrogenated maltose starch syrup and 40 g of macrogol 400 were dissolved in 500 g of water. Then, to this solution were added a solution obtained by dissolving 25 g of 1-menthol in 250 g of anhydrous ethanol and a solution obtained by dispersing 50 g of titanium oxide in 500 g of anhydrous ethanol. Then, 335 g of hypromellose was added to the resulting solution to obtain a solution for preparing a coating layer.

27 g of loperamide hydrochloride, 64.8 g of macrogol 400, 256.5 g of powdered hydrogenated maltose starch syrup, and 54 g of saccharin sodium were dissolved in a mixture solution of 810 g of water and 810 g of anhydrous ethanol. To this mixture solution were added 407.7 g of hydroxypropylcellulose and 1.35 g of a flavoring agent to obtain a solution for preparing a drug-containing intermediate layer.

The solution for preparing a coating layer was uniformly applied on a PET film and then dried with hot air to form a coating layer with a weight of 5 mg per area of 2.72 cm². The solution for preparing a drug-containing intermediate layer was uniformly applied on the upper side of the coating layer and then dried with hot air to form a drug-containing intermediate layer with a weight of 7.5125 mg per area of 2.72 cm² as Interim Product a.

Two sets of Interim Product a were prepared, contacted so that the drug-containing intermediate layers are opposed to each other, and bonded by pressure, to obtain Interim Product b in which the coating layer, the drug-containing intermediate layer and the coating layer were laminated in this order between the two PET films.

One PET film of Interim Product b was peeled off, the product was cut into an area of 2.72 cm², and the other PET film was peeled off to obtain a film preparation.

### Comparative example 2

50 g of powdered hydrogenated maltose starch syrup and 40 g of macrogol 400 were dissolved in 500 g of water. Then, to this solution were added a solution obtained by dissolving 25 g of 1-menthol in 250 g of anhydrous ethanol and a solution obtained by dispersing 50 g of titanium oxide in 500 g of anhydrous ethanol. Then, 335 g of hypromellose was added to the resulting solution to obtain a solution for preparing a coating layer.

27 g of loperamide hydrochloride, 64.8 g of macrogol 400, 256.5 g of powdered hydrogenated maltose starch syrup, 54 g of saccharin sodium, and 27 g of 1-menthol were dissolved in a mixture solution of 810 g of water and 810 g anhydrous ethanol. 380.7 g of hydroxypropylcellulose and 1.35 g of flavoring agent were added to this mixture solution to obtain a solution for preparing a drug-containing intermediate layer.

The solution for preparing a coating layer was uniformly applied on a PET film and then dried with hot air to form a coating layer with a weight of 5 mg per area of 2.72 cm². The solution for preparing a drug-containing intermediate layer was uniformly applied on the upper side of the coating layer and then dried with hot air to form a drug-containing intermediate layer with a weight of 7.5125 mg per area of 2.72 cm² as Interim Product c.

Two sets of Interim Product c were prepared, contacted so that the drug-containing intermediate layers are opposed to each other, and bonded by pressure, to obtain Interim Product d in which the coating layer, the drug-containing intermediate layer and the coating layer were laminated in this order between the two PET films.

One PET film of Interim Product d was peeled off, the product was cut into an area of 2.72 cm², and the other PET film was peeled off to obtain a film preparation.

### Test Example

### Bitter taste masking test

The film preparations obtained in Example 1, Comparative Example 1, and Comparative Example 2 were evaluated for sensation when they are taken. A sensory test was conducted, in which the film preparations were evaluated by six panelists for sensation when they are taken (bitter taste). The evaluation criteria were "a bitter taste is virtually unnoticeable" given 2 points, "a bitter taste is slightly noticeable" given 1 point, and "a bitter taste is noticeable and the taste is bad" given 0 point. Accordingly, a higher score indicates that a bitter taste is more masked. The total scores are shown in Table 1, with the composition of each film preparation.

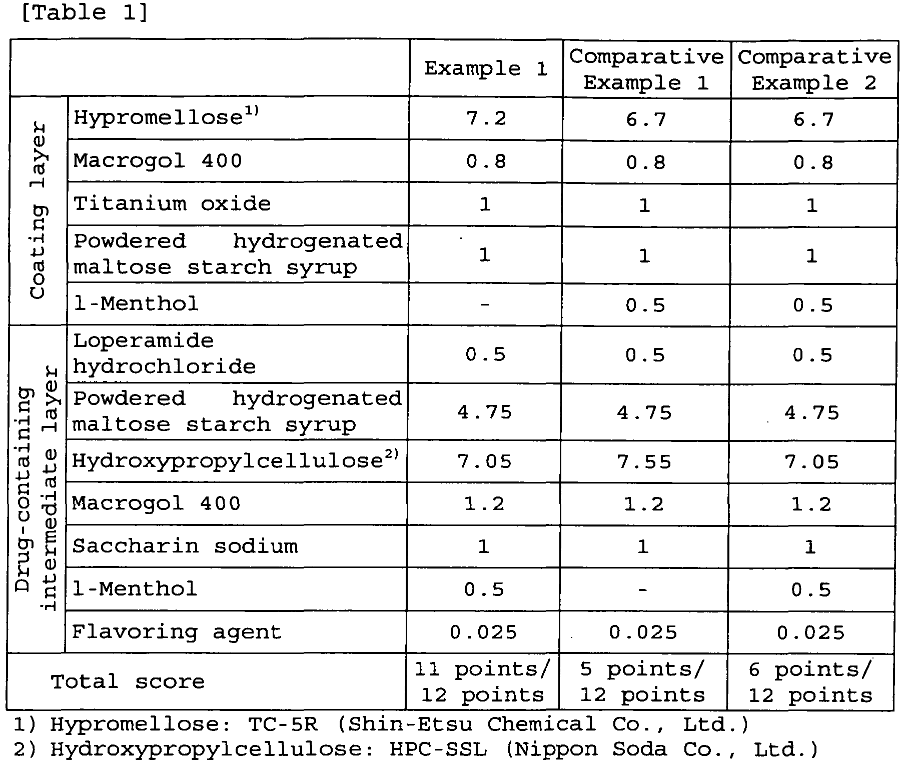

As shown in Table 1, it was confirmed that, the film preparation of the present invention (Example 1) with a drug-containing intermediate layer containing loperamide hydrochloride having a strong bitter taste together with menthol, which was sandwiched between coating layers not containing menthol could mask the bad taste of loperamide hydrochloride and a film preparation with an easy-to-take could be produced.

On the other hand, the film preparation of Comparative Example 1 with a drug-containing intermediate layer containing loperamide hydrochloride but not menthol, which was sandwiched between coating layers containing menthol could not mask the bad taste of loperamide hydrochloride. Furthermore, the film preparation of Comparative Example 2 with a drug-containing intermediate layer containing loperamide hydrochloride and menthol, which was sandwiched between coating layers containing menthol could not mask the bad taste loperamide hydrochloride either.

Accordingly, it was found that masking of the bad taste of loperamide hydrochloride could not be achieved simply by adding menthol and masking of a bad taste could be achieved by adding menthol to only a drug-containing intermediate layer.

Furthermore, when the film preparation of the present invention (Example 1) was taken without water, it was dissolved in the oral cavity within 30 seconds. It was therefore confirmed that a fast-acting property of the film preparation could be expected.

## Claims

1. A film preparation comprising: a drug-containing intermediate layer containing loperamide hydrochloride, a terpene and a film-forming agent; and coating layers containing a film-forming agent and a plasticizer (but not a terpene) and laminated on both sides of the drug-containing intermediate layer.

2. The film preparation according to claim 1, wherein a dose of loperamide hydrochloride for one treatment is 0.5 to 2 mg for adults.

3. The film preparation according to claim 1 or 2, wherein a content of loperamide hydrochloride in the film preparation is 0.1% to 15% by weight to the whole film preparation.

4. The film preparation according to any one of claims 1 to 3, wherein a content of a terpene in the film preparation is 0.2% to 15% by weight to the whole film preparation.

5. The film preparation according to any one of claims 1 to 4, wherein a content of a terpene is a nearly equal amount to that of loperamide hydrochloride.

6. The film preparation according to any one of claims 1 to 5, wherein the terpene is menthol.

7. The film preparation according to any one of claims 1 to 6, wherein the film-forming agent is hydroxypropylcellulose and/or hypromellose.

8. The film preparation according to any one of claims 1 to 7, which is dissolved in the oral cavity.

9. The film preparation according to any one of claims 1 to 8, wherein a bitter taste derived from loperamide hydrochloride is masked.

10. A method for masking a bitter taste in a film preparation containing loperamide hydrochloride, comprising
laminating coating layers containing a film-forming agent and a plasticizer (but not a terpene) on both sides of a drug-containing intermediate layer containing loperamide hydrochloride, a terpene and a film-forming agent.

11. A method for manufacturing a loperamide hydrochloride containing film preparation, wherein a bitter taste derived from loperamide hydrochloride is masked, comprising
laminating coating layers containing a film-forming agent and a plasticizer (but not a terpene) on both sides of a drug-containing intermediate layer containing loperamide hydrochloride, a terpene and a film-forming agent.

## Patentansprüche

1. Filmpräparat, umfassend eine Arzneimittel-haltige Zwischenschicht, die Loperamidhydrochlorid, ein Terpen und ein Film-bildendes Mittel enthält; und Deckschichten, die ein Film-bildendes Mittel und einen Weichmacher (jedoch kein Terpen) enthalten und auf beide Seiten der Arzneimittel-haltigen Zwischenschicht laminiert sind.

2. Filmpräparat nach Anspruch 1, wobei die Dosis von Loperamidhydrochlorid für eine Behandlung 0,5 bis 2 mg für Erwachsene beträgt.

3. Filmpräparat nach Anspruch 1 oder 2, wobei der Gehalt an Loperamidhydrochlorid in dem Filmpräparat 0,1 bis 15 Gewichtsprozent bezogen auf das gesamte Filmpräparat beträgt.

4. Filmpräparat nach einem der Ansprüche 1 bis 3, wobei der Gehalt an Terpen in dem Filmpräparat 0,2 bis 15 Gewichtsprozent bezogen auf das gesamte Filmpräparat beträgt.

5. Filmpräparat nach einem der Ansprüche 1 bis 4, wobei der Gehalt an Terpen die annähernd gleiche Menge wie die des Loperamidhydrochlorids ist.

6. Filmpräparat nach einem der Ansprüche 1 bis 5, wobei das Terpen Methanol ist.

7. Filmpräparat nach einem der Ansprüche 1 bis 6, wobei das Film-bildende Mittel Hydroxypropylcellulose und/oder Hypromellose ist.

8. Filmpräparat nach einem der Ansprüche 1 bis 7, welches in der Mundhöhle aufgelöst wird.

9. Filmpräparat nach einem der Ansprüche 1 bis 8, wobei der bittere Geschmack, der von dem Loperamidhydrochlorid stammt, maskiert wird.

10. Verfahren zum Maskieren des bitteren Geschmacks in einem Loperamidhydrochlorid enthaltenden Filmpräparat, umfassend das Laminieren von Deckschichten, die ein Film-bildendes Mittel und einen Weichmacher (jedoch kein Terpen) enthalten, auf beide Seiten einer Arzneimittel-haltigen Zwischenschicht, die Loperamidhydrochlorid, ein Terpen und ein Film-bildendes Mittel enthält.

11. Verfahren zum Herstellen eines Loperamidhydrochlorid enthaltenden Filmpräparats, wobei der bittere Geschmack, der von dem Loperamidhydrochlorid stammt, maskiert wird, umfassend das Laminieren von Deckschichten, die ein Film-bildendes Mittel und einen Weichmacher (jedoch kein Terpen) enthalten, auf beide Seiten einer Arzneimittel-haltigen Zwischenschicht, die Loperamidhydrochlorid, ein Terpen und ein Film-bildendes Mittel enthält.

## Revendications

1. Préparation de film comprenant : une couche intermédiaire contenant un médicament, qui contient du chlorhydrate de lopéramide, un terpène et un agent filmogène ; et des couches de revêtement contenant un agent filmogène et un plastifiant (mais pas un terpène) et stratifiées sur les deux côtés de la couche intermédiaire contenant un médicament.

2. Préparation de film selon la revendication 1, dans laquelle la dose de chlorhydrate de lopéramide pour un traitement est de 0,5 à 2 mg pour des adultes.

3. Préparation de film selon la revendication 1 ou 2, dans laquelle la teneur en chlorhydrate de lopéramide de la préparation de film est de 0,1 % à 15 % en poids de la préparation de film totale.

4. Préparation de film selon l'une quelconque des revendications 1 à 3, dans laquelle la teneur en terpène de la préparation de film est de 0,2 % à 15 % en poids de la préparation de film totale.

5. Préparation de film selon l'une quelconque des revendications 1 à 4, dans laquelle la teneur en terpène est une quantité pratiquement égale à celle du chlorhydrate de lopéramide.

6. Préparation de film selon l'une quelconque des revendications 1 à 5, dans laquelle le terpène est le menthol.

7. Préparation de film selon l'une quelconque des revendications 1 à 6, dans laquelle l'agent filmogène est l'hydroxypropylcellulose et/ou l'hypromellose.

8. Préparation de film selon l'une quelconque des revendications 1 à 7, qui est dissoute dans la cavité orale.

9. Préparation de film selon l'une quelconque des revendications 1 à 8, dans laquelle le goût amer dérivé du chlorhydrate de lopéramide est masqué.

10. Procédé pour masquer un goût amer dans une préparation de film contenant du chlorhydrate de lopéramide, comprenant la stratification de couches de revêtement contenant un agent filmogène et un plastifiant (mais pas un terpène) sur les deux côtés d'une couche intermédiaire contenant un médicament qui contient du chlorhydrate de lopéramide, un terpène et un agent filmogène.

11. Procédé pour fabriquer une préparation de film contenant du chlorhydrate de lopéramide, dans lequel le goût amer dérivé du chlorhydrate de lopéramide est masqué, comprenant la stratification de couches de revêtement contenant un agent filmogène et un plastifiant (mais pas un terpène) sur les deux côtés d'une couche intermédiaire contenant un médicament qui contient du chlorhydrate de lopéramide, un terpène et un agent filmogène.
